# EUROPÄISCHE PATENTANMELDUNG

(11) **EP 2 462 916 A2**
(43) Veröffentlichungstag der Anmeldung: **13.06.2012**
(21) Anmeldenummer: 11188455.7
(22) Anmeldetag: 09.11.2011
(51) Int. Cl.: A61K 8/19, A61K 8/27, A61K 8/34, A61K 8/37, A61K 8/49, A61Q 11/00

(54) **Mund- und Zahnpflege- und -Reinigungsmittel mit gesteigerter antibakterieller Wirkung I**

(30) Priorität: 08.12.2010 DE 102010062610
(71) Anmelder: Henkel AG & Co. KGaA, 40589 Düsseldorf (DE)
(72) Erfinder: Adomat, Christel, 40591 Düsseldorf (DE); Banowski, Bernhard, 40597 Düsseldorf (DE); Breves, Roland, 40822 Mettmann (DE); Simmering, Rainer, 41515 Grevenbroich (DE)

(57) **Zusammenfassung**

Die antiinflammatorische und vorbeugende Wirkung antimikrobieller Substanzen lässt sich überraschenderweise durch den Einsatz von 4-(3-Ethenyl-3,7-dimethyl-1,6-octadienyl)-phenol steigern. Entsprechende vorteilhafte Mittel sind insbesondere Mund- und Zahnpflege-und -reinigungsmittel, die - bezogen auf ihr Gewicht - 0,001 bis 5 Gew.-% 4-(3-Ethenyl-3,7-dimethyl-1,6-octadienyl)-phenol und 0,00001 bis 5 Gew.-% mindestens einer antibakteriellen Verbindung aus den Gruppen der Benzoate und/oder der Parabene und/oder der Cu-Salze und/oder der Ag-Salze und/oder der Zn-Salze und/oder Triclosan und/oder Hexetidin enthalten.

## Beschreibung

Die Erfindung betrifft Zubereitungen zur Mund- und Zahnpflege- und -reinigung, die aufgrund von speziellen Inhaltsstoffen eine ausgezeichnete zahnfleischpflegende und -erhaltende Wirkung besitzen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u.a. flüchtige Schwefelverbindungen ("volatile sulfur compounds", VSC) entstehen, die für den üblen Geruch verantwortlich gemacht werden.

Diese Schwefelverbindungen sind insbesondere: H₂S = Schwefelwasserstoff (zu etwa 30 %), CH₃-S-H = Methylmercaptan (zu etwa 60 %) und CH₃-S-CH₃ = Dimethylsulfid (zu etwa 10 %).

Die flüchtigen Schwefelverbindungen sind teilweise sehr aggressiv und können das Zahnfleisch und die Mundschleimhaut schädigen. So ist beispielsweise bekannt, daß Schwefelwasserstoff und Methylmercaptan die Durchlässigkeit der Mundschleimhaut erhöhen und so Zahnfleischerkrankungen Vorschub leisten können.

Zahnfleischerkrankungen stellen einen hohen Risikofaktor für die Gesundheit dar. Nach Untersuchungen der Weltgesundheitsorganisation (WHO) leiden über 50 % der erwachsenen Bundesbürger an dringend behandlungsbedürftigen Parodontopathien. Unter Parodontitis versteht man eine durch Bakterien hervorgerufene entzündliche Veränderung des den Zahn umgebenden Gewebes, besonders des Kieferknochens. Eine Parodontitis (= mit Beteiligung der Zahnfleischtaschen und des Knochens) entwickelt sich immer aus einer Zahnfleischentzündung (Gingivitis) und befällt zuerst die der Reinigung am schlechtesten zugänglichen Zahnzwischenräume. Die sich in der Zahnfleischtasche befindlichen Bakterien wirken durch ihre Stoffwechselprodukte, insbesondere durch die von Ihnen produzierten VSC, destruktiv auf Zahnfleisch, Zahnsubstanz und Knochen ein und verstärken so ständig die bereits bestehende Parodontitis.

Es hat nicht an Versuchen gefehlt, Mundgeruch und entzündliche Zustände in der Mundhöhle zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung. Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können. In Einzelfällen kann die Bekämpfung der Bakterien nicht ausreichend sein, um eine Rückgang des entzündlichen Zustandes zu erreichen.

Es besteht daher nach wie vor das Bedürfnis, Halitosis zu bekämpfen und Wirkstoffe oder Wirkstoffkombinationen dafür bereitzustellen, die frei von den genannten Nachteilen sind.

Die Verwendung von Pflanzenextrakten, welche 4-(3-Ethenyl-3,7-dimethyl-1,6-octadienyl)-phenol enthalten, in Produkten zur oralen Hygiene wird in der JP 2000 327581 beschrieben.

Die Aufgabe der vorliegenden Erfindung bestand darin, Zubereitungen zur Mund- und Zahnpflege und -reinigung bereitzustellen, die Mundgeruch wirksam bekämpfen und gegen Gingivitis und Parodontitis wirksam sind. Dabei sollten in einem Aspekt der Erfindung die Nachteile des Einsatzes von Triclosan, Zinnsalzen oder Chlorhexidin vermieden, deren Einsatzmenge verringert oder auf natürliche antimikrobielle Stoffe zurückgegriffen werden können. In einem weiteren Aspekt der Erfindung sollte die Pflege des Mundraumes durch die Unterstützung der natürlichen Regeneration der Mundschleimhaut und des Zahnfleisches verbessert werden.

Es wurde nun gefunden, daß sich bestimmte Inhaltsstoffe zur Lösung des vorstehend genannten Aufgabenkomplexes eignen.

Gegenstand der vorliegenden Erfindung ist in einer ersten Ausführungsform ein Mund- und Zahnpflege- und -reinigungsmittel, enthaltend - bezogen auf sein Gewicht -
a) 0,001 bis 5 Gew.-% 4-(3-Ethenyl-3,7-dimethyl-1,6-octadienyl)-phenol und
b) 0,00001 bis 5 Gew.-% mindestens einer antibakteriellen Verbindung aus den Gruppen
   a. der Benzoate und/oder
   b. der Parabene und/oder
   c. der Cu-Salze und/oder
   d. der Ag-Salze und/oder
   e. der Zn-Salze und/oder
   f. Triclosan und/oder
   g. Hexetidin.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Zusammensetzungen bezogen auf ihr Gewicht 0,001 bis 5 Gew.-% 4-(3-Ethenyl-3,7-dimethyl-1,6-octadienyl)-phenol. Bevorzugte Zusammensetzungen enthalten 4-(3-Ethenyl-3,7-dimethyl-1,6-octadienyl)-phenol innerhalb engerer Grenzen. Hier sind bevorzugte erfindungsgemäße Mund- und Zahnpflege-und -reinigungsmittel dadurch gekennzeichnet, daß sie - bezogen auf ihr Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% 4-(3-Ethenyl-3,7-dimethyl-1,6-octadienyl)-phenol enthalten.

4-(3-Ethenyl-3,7-dimethyl-1,6-octadienyl)-phenol (gelegentlich auch als "Bakuchiol" bezeichnet) wird aus Samen von Psoralea corylifolia, einem in China heimischen Baum aus der Familie Fabaceae isoliert, der vielfältige Anwendung in der traditionellen chinesischen Medizin sowie im indischen Ayurveda findet. Die Samen enthalten verschiedene Coumarine einschließlich Psoralen. 4-(3-Ethenyl-3,7-dimethyl-1,6-octadienyl)-phenol läßt sich durch folgende Formel beschreiben:

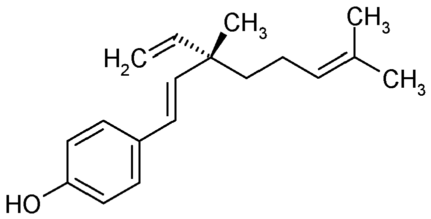

Die erfindungsgemäßen Mittel enthalten das antibakteriell und antimykotisch wirkende 4-(3-Ethenyl-3,7-dimethyl-1,6-octadienyl)-phenol in Kombination mit einem weiteren antibakteriellen Mittel, dessen Wirkung in dieser Kombination synergistisch gesteigert wird. Dadurch ist es möglich, die Einsatzmengen antimikrobieller Verbindungen insgesamt zu verringern.

Eine erfindungsgemäß bevorzugte Gruppe antimikrobieller Verbindungen, die zusätzlich zum 4-(3-Ethenyl-3,7-dimethyl-1,6-octadienyl)-phenol in den erfindungsgemäßen Mitteln eingesetzt werden kann, sind die Benzoate. In Mund und Zahnpflege- und -reinigungsmitteln können beispielsweise Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218) oder Methyl-4-hydroxybenzoat, Natriumsalz (E 219) eingesetzt werden. Ganz besonders bevorzugt ist der Einsatz von Natriumbenzoat und/oder p-Hydroxybenzoesäureestern, wobei besonders bevorzugte erfindungsgemäße Mund- und Zahnpflege- und reinigungsmittel dadurch gekennzeichnet sind, daß sie - bezogen auf ihr Gewicht - 0,01 - 4 Gew.-%, vorzugsweise 0,02 - 3 Gew.-%, besonders bevorzugt 0,05 - 2 Gew.-%, außerordentlich bevorzugt 0,1 - 1,5 Gew.-% und insbesondere 0,1 bis 1,0 Gew.-% p-Hydroxybenzoesäuremethyl-, -ethyl- oder propylester oder deren Mischungen enthalten.

Ebenfalls bevorzugte erfindungsgemäße Mund- und Zahnpflege- und reinigungsmittel enthalten - bezogen auf ihr Gewicht - 0,01 - 4 Gew.-%, vorzugsweise 0,02 - 3 Gew.-%, besonders bevorzugt 0,05 - 2 Gew.-%, außerordentlich bevorzugt 0,1 - 1,5 Gew.-% und insbesondere 0,1 bis 1,0 Gew.-% Natriumbenzoat.

Eine weitere erfindungsgemäß bevorzugte Gruppe antimikrobieller Verbindungen, die zusätzlich zu oder anstelle von Benzoaten in den erfindungsgemäßen Mitteln eingesetzt werden kann, sind die Parabene. Erfindungsgemäß bevorzugte Mund- und Zahnpflege- und -reinigungsmittel enthalten - bezogen auf ihr Gewicht - 0,01 - 4 Gew.-%, vorzugsweise 0,02 - 3 Gew.-%, besonders bevorzugt 0,05-2 Gew.-%, außerordentlich bevorzugt 0,1 - 1,5 Gew.-% und insbesondere 0,1 bis 1,0 Gew.-% Parabene, wobei besonders bevorzugte erfindungsgemäße Mund- und Zahnpflege-und -reinigungsmittel dadurch gekennzeichnet sind, daß sie - bezogen auf ihr Gewicht - 0,01 - 4 Gew.-%, vorzugsweise 0,02 - 3 Gew.-%, besonders bevorzugt 0,05 - 2 Gew.-%, außerordentlich bevorzugt 0,1 - 1,5 Gew.-% und insbesondere 0,1 bis 1,0 Gew.-% Parabene aus der Gruppe Methylparaben, Ethylparaben und deren Mischungen enthalten.

Eine weitere erfindungsgemäß bevorzugte Gruppe antimikrobieller Verbindungen, die zusätzlich zu oder anstelle von Benzoaten und/oder Parabenen in den erfindungsgemäßen Mitteln eingesetzt werden kann, sind die Cu-Salze.

Als Kupferionen liefernde Verbindungen sind prinzipiell alle toxikologisch unbedenklichen, schleimhautverträglichen Kupferverbindungen geeignet. Von den anorganischen Salzen seien beispielhaft genannt: Kupferchlorid, CuCl₂, und dessen Dihydrat; Kupferfluorid CuF₂, und dessen Dihydrat; Kupferfluosilikat, CuSiF₆, und dessen Hexahydrat; Kupfersulfat, CuSO₄, und dessen Pentahydrat; Kupfernitrat bzw. dessen Tri- und Hexahydrat sowie auch ausgefallenere Kupfersalze wie Bromide, Bromate, Chlorate, Jodate, Fluorphosphate. Bevorzugte Kupfersalze organischer Säuren sind das Acetat, Formiat, Benzoat, Citrat, Tartrat, Lactat, Malat, Mandelat, Sorbat, Pantothenat, Gluconat, Phytat, Glycerophosphat, Cinnamat, Butyrat, Propionat, Laurat, Oxalat, Salicylat. Geeignet sind auch die Kupfersalze von Aminosäuren wie Glycinat oder Glutamat. Ein besonders bevorzugtes Aminosäuresalz ist das Kupferaspartat.

Unabhängig von der Art der Kupfersalze werden diese vorzugsweise in solchen Mengen eingesetzt, daß eine bestimmte Kupferionenkonzentration im Mittel gewährleistet ist. Bevorzugte erfindungsgemäße Mund- und Zahnpflege- und -reinigungsmittel enthalten - bezogen auf ihr Gewicht - 0,00001 - 0,1 Gew.-%, vorzugsweise 0,00002 - 0,05 Gew.-%, besonders bevorzugt 0,00005 - 0,025 Gew.-%, außerordentlich bevorzugt 0,000075 - 0,015 Gew.-% und insbesondere 0,0001 bis 0,001 Gew.-% Kupferionen, berechnet als Cu²⁺.

Eine weitere erfindungsgemäß bevorzugte Gruppe antimikrobieller Verbindungen, die zusätzlich zu oder anstelle von Benzoaten und/oder Parabenen und/oder Cu-Salzen in den erfindungsgemäßen Mitteln eingesetzt werden kann, sind die Ag-Salze. Geeignete Silbersalze sind beispielsweise Silbernitrat, Silberacetat, Silberlactat, Silberphosphat, Silberchlorid, Silberbromid, Silberhydroxid, Silbercarbonat, Silberoxid, Silberperiodat oder der Natriumchlorid-Silberchloridkomplex (Na[AgCl ₂ ]). Unabhängig von der Art der Silbersalze werden diese vorzugsweise in solchen Mengen eingesetzt, daß eine bestimmte Silberionenkonzentration im Mittel gewährleistet ist. Bevorzugte erfindungsgemäße Mund- und Zahnpflege- und -reinigungsmittel enthalten - bezogen auf ihr Gewicht - 0,00001 - 0,1 Gew.-%, vorzugsweise 0,00002 - 0,05 Gew.-%, besonders bevorzugt 0,00005 - 0,025 Gew.-%, außerordentlich bevorzugt 0,000075 - 0,015 Gew.-% und insbesondere 0,0001 bis 0,001 Gew.-% Silberionen, berechnet als Ag⁺.

Eine weitere erfindungsgemäß bevorzugte Gruppe antimikrobieller Verbindungen, die zusätzlich zu oder anstelle von Benzoaten und/oder Parabenen und/oder Cu-Salzen und/oder Ag-Salzen in den erfindungsgemäßen Mitteln eingesetzt werden kann, sind die Zn-Salze.

Erfindungsgemäß können sowohl anorganische als auch organische Salze des Zinks eingesetzt werden.

Neben den nicht löslichen anorganischen Zinksalzen, also Salzen, welche eine Löslichkeit unterhalb 100 mg/L (20°C), vorzugsweise unterhalb 10 mg/L (20°C), insbesondere keine Löslichkeit (20°C) aufweisen (Bsp.: Zinkoxid), sind im Rahmen der vorliegenden Anmeldung die löslichen anorganischen Zinksalze, das heißt Salze, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen, bevorzugter Bestandteil erfindungsgemäßer Mittel. Zu den bevorzugten löslichen anorganischen Salzen zählen das Zinkbromid, das Zinkchlorid, das Zinkiodid, das Zinknitrat und das Zinksulfat.

Erfindungsgemäß besonders bevorzugte Mittel enthalten dabei mindestens ein Zinksalz aus der nachstehenden Tabelle:

| Zinksalz | Löslichkeit |
|---|---|
| Zinkacetat-Dihydrat | 430 g/l (20°C) |
| Zin kacetylacetonat | 4 g/l (20°C) |
| Zinkbromid | 820 g/l (25°C) |
| Zinkchlorid | 4320 g/l (25°C) |
| Zinkgluconat | 100 g/l (20°C) |
| Zinkhydroxycarbonat | Fast unlöslich (20°C) |
| Zinkiodid | 4500 g/l (20°C) |
| Zinknitrat Hexahydrat | 1843 g/l (20°C) |
| Zinknitrat-Tetrahydrat | Leicht löslich (20°C) |
| Zinkoxid | Unlöslich |
| Zinkstearat | 0,9 mg/l (20°C) |
| Zinksulfat-Heptahydat | 960 g/l (20°C) |
| Zinksulfat-Monohydrat | ~350 g/l (20°C) |

Das Spektrum der erfindungsgemäß bevorzugten Zinksalze organischer Säuren, vorzugsweise organischer Carbonsäuren, reicht von Salzen die in Wasser nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinlaureat, das Zinkoleat, das Zinkoxalat, das Zinktartrat und das Zinkstearat, zu der Gruppe der löslichen organischen Zinksalze gehören beispielsweise das Zinkacetat, das Zinkacetylacetonat, das Zinkbenzoat, das Zinkformiat, das Zinklactat, das Zinkgluconat, das Zinkvalerat sowie das Zinksalz der p-Toluolsulfonsäure.

Es hat sich gezeigt, daß Zinksulfat ein besonders bevorzugt einzusetzendes Zinksalz ist. Zinksulfat bildet mehrere Hydrate. Das Heptahydrat bildet sich aus gesättigter wäßriger Lösung in Form farbloser, glasglänzender, säulenförmiger, rhombischer Kristalle. Oberhalb 39°C erfolgt Umwandlung zu ZnSO₄ · 6H₂O, und bei 70°C liegt nur noch ZnSO₄ · H₂O vor; das letzte H₂O-Molekül entweicht bei 240°C. Interessanterweise steigt die erfindungsgemäße Wirkung bei den Zinksulfaten mit deren Kristallwassergehalt, so daß das Heptahydrat eine besonders bevorzugte Verbindung ist.

Erfindungsgemäß bevorzugte Mund- und Zahnpflege- und -reinigungsmittel enthalten daher Zinksulfat, vorzugsweise Zinksulfat-Heptahydrat.

Unabhängig von der Art der Zinksalze werden diese vorzugsweise in solchen Mengen eingesetzt, daß eine bestimmte Zinkionenkonzentration im Mittel gewährleistet ist. Bevorzugte erfindungsgemäße Mund- und Zahnpflege- und -reinigungsmittel enthalten - bezogen auf ihr Gewicht - 0,01 - 5 Gew.-%, vorzugsweise 0,02 - 2,5 Gew.-%, besonders bevorzugt 0,03 - 1 Gew.-%, außerordentlich bevorzugt 0,04 - 0,5 Gew.-% und insbesondere 0,05 bis 0,2 Gew.-% Zinkionen, berechnet als Zn²⁺.

Eine weitere erfindungsgemäß bevorzugte antimikrobielle Verbindung, die zusätzlich zu oder anstelle von Benzoaten und/oder Parabenen und/oder Cu-Salzen und/oder Ag-Salzen und/oder Zn-Salzen in den erfindungsgemäßen Mitteln eingesetzt werden kann, ist Triclosan.

Triclosan (2,4,4'-Trichlor-2'-hydroxydiphenylether) wird vorzugsweise innerhalb engerer Mengenbereiche eingesetzt, so daß bevorzugte erfindungsgemäße Mund- und Zahnpflege- und - reinigungsmittel - bezogen auf sein Gewicht - 0,05 - 0,75 Gew.-%, vorzugsweise 0,06 - 0,5 Gew.-%, besonders bevorzugt 0,07 - 0,4 Gew.-%, außerordentlich bevorzugt 0,08 - 0,35 Gew.-% und insbesondere 0,1 bis 0,3 Gew.-% Triclosan enthalten.

Eine weitere erfindungsgemäß bevorzugte antimikrobielle Verbindung, die zusätzlich zu oder anstelle von Benzoaten und/oder Parabenen und/oder Cu-Salzen und/oder Ag-Salzen und/oder Zn-Salzen und/oder Tricolsan in den erfindungsgemäßen Mitteln eingesetzt werden kann, ist Hexetidin.

Hexetidin, auch bezeichnet als 5-Amino-1,3-bis(2-ethylhexal)hexahydro-5-methylpyrimidin oder 1,3-Bis(2-ethylhexyl)-hexahydro-5-methylpyrimidin-5-amin ist ein Antiseptikum bzw. Desinfektionsmittel, das durch folgende Formel wiedergegeben wird:

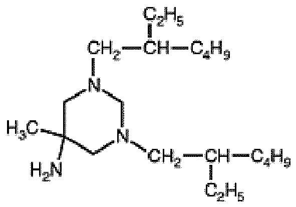

In den erfindungsgemäßen Mitteln wird Hexetidin vorzugsweise ebenfalls innerhalb engerer Mengenbereiche eingesetzt, so daß erfindungsgemäß bevorzugte Mund- und Zahnpflege-und -reinigungsmittel - bezogen auf ihr Gewicht - 0,01 - 0,5 Gew.-%, vorzugsweise 0,02 - 0,4 Gew.-%, besonders bevorzugt 0,03 - 0,3 Gew.-%, außerordentlich bevorzugt 0,04 - 0,2 Gew.-% und insbesondere 0,05 bis 0,1 Gew.-% 1,3-Bis(2-ethylhexyl)-hexahydro-5-methylpyrimidin-5-amin (Hexetidin) enthalten.

Die erfindungsgemäßen Mund- und Zahnpflege- und -reinigungsmittel enthalten weitere Inhaltsstoffe. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthalten, sind erfindungsgemäß bevorzugt.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Der Einsatz von Putzkörpern (Abrasiva) ist ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®} 12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 --250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel können z.B. auch Substanzen enthalten, die gegen Plaque und/oder Zahnstein wirksam sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside sowie Fettsäureamidoalkylbetaine.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten. Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Es hat sich gezeigt, daß die erfindungsgemäßen Mund- und Zahnpflege- und -reinigungsmittel in ihrer Leistung weiter gesteigert werden können, wenn die Mittel salivationsfördernde Substanzen enthalten. Insbesondere die antibakterielle Wirkung und mit ihr die Antikarieswirkung und die Wirkung gegen Gingivitis und/oder Parodontitis werden hierdurch verstärkt.

Unter Salivation versteht man die Speichelproduktion und -freisetzung, im weiteren Sinne auch in unphysiologisch erhöhter Menge. Substanzen, die den Speichelfluß anregen und die Speichelmenge und/oder -freisetzung erhöhen, können aus den unterschiedlichsten Stoffklassen stammen.

Eine erfindungsgemäß beispielsweise geeignete Substanz ist das Pilocarpin, das in den erfindungsgemäßen Mund- und Zahnpflege- und -reinigungsmitteln enthalten sein kann.

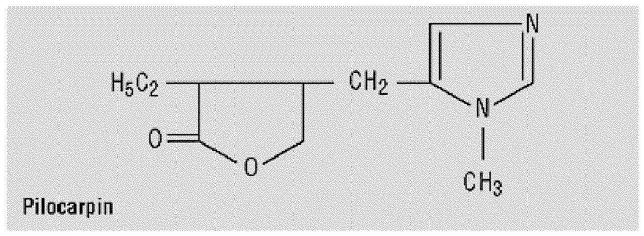

Weitere salivationsfördernde Substanzen sind insbesondere sogenannte Scharfstoffe, d.h. scharf schmeckende und/oder ein Gefühl von Wärme erzeugende Substanzen. Erfindungsgemäß bevorzugte Mund- und Zahnpflege- und -reinigungsmittel sind dadurch gekennzeichnet, daß sie als salivationsfördernde Substanz mindestens eine scharf schmeckende und/oder ein Gefühl von Wärme erzeugende Substanz enthalten.

Als salivationsfördernden Inhaltsstoff enthalten die erfindungsgemäßen Erzeugnisse dieser Ausführungsform eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor und fördern dadurch den Speichelfluß.

Erfindungsgemäß bevorzugte Erzeugnisse dieser Ausführungsform enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.

Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid
- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid
- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid
- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid
- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid
- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid
- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid
- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid
- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid
- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid
- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid
- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt): 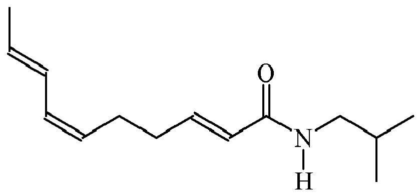
2E,4E,8Z-Decatriensäure-N-isobutylamid (N-Isobutyl-2E,4E,8Z-decatrienamid, auch Isoaffinin genannt): 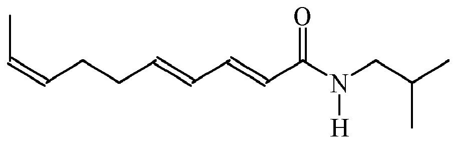
2E,7Z,9E-Undecatriensäure-N-isobutylamid (N-Isobutyl-2E,7Z,9E-undecatrienamid): 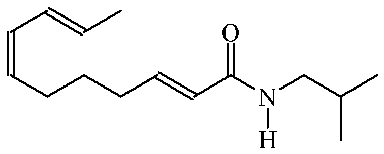
2E,4Z-Decadiensäure-N-isobutylamid (cis-Pellitorin): 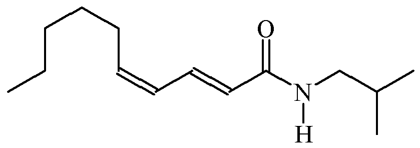
2E,4E-Decadiensäure-N-isobutylamid (trans-Pellitorin):

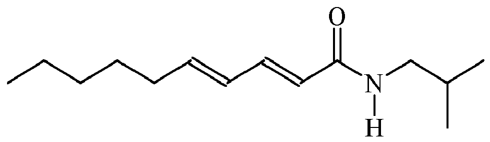

Ferulasäureamide, beispielsweise

Ferulasäure-N-Vanillylamid:

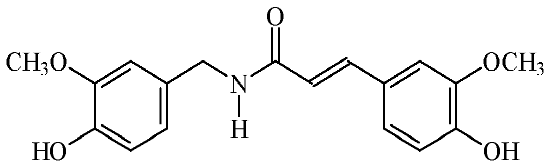

*N*-[2-(4-Hydroxy-3-methoxyphenyl)ethyl]-3-(4-hydroxy-3-methoxy-phenyl)-(2*E*)-propensäureamid (trans-Feruloylmethoxytyramin):

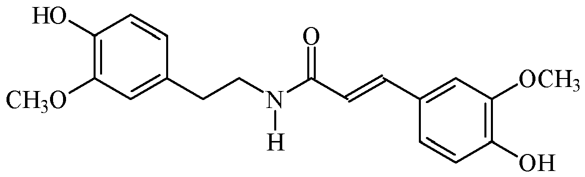

*N*-[2-(4-Hydroxy-3-methoxyphenyl)ethyl]-3-(4-hydroxy-3-methoxy-phenyl)-(2*Z*)-propensäureamid (cis-Feruloylmethoxytyramin):

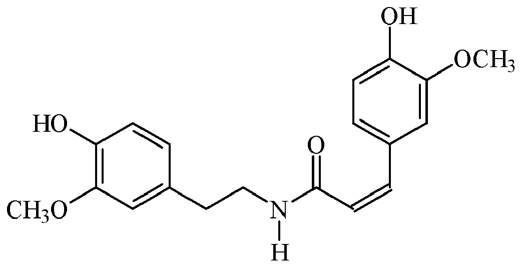

*N*-[2-(4-Hydroxy-3-methoxyphenyl)ethyl]-3-(4-hydroxy-3-methoxy-phenyl)-propansäureamid (Dihydroferuloylmethoxytyramin):

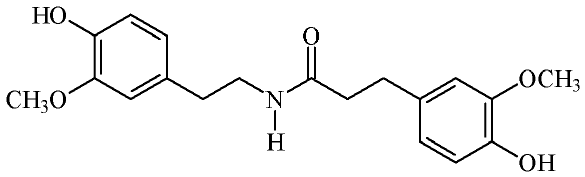

*N*-[2-(3,4- Dihydroxyphenyl)ethyl]-3-(4-hydroxy-3-methoxy-phenyl)-(2*E*)-propensäureamid (trans-Feruloyldopamin):

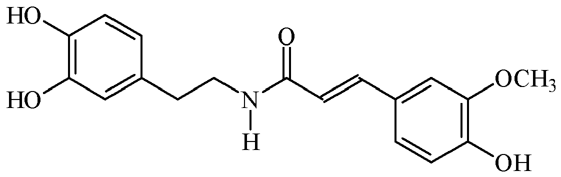

*N*-[2-(3,4-Dihydroxyphenyl)ethyl]-3-(4-hydroxy-3-methoxy-phenyl)-(2*Z*)-propensäureamid (cis-Feruloyldopamin):

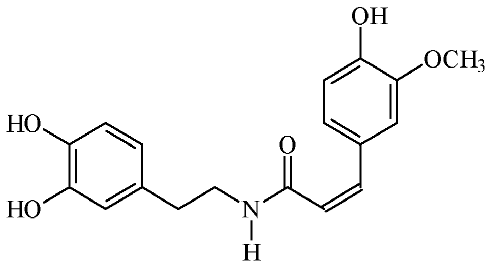

*N*-[2-(4-Hydroxyphenyl)ethyl]-3-(3,4-dihydroxyphenyl)-(2*E*)-propensäureamid (trans-Caffeoyltyramin):

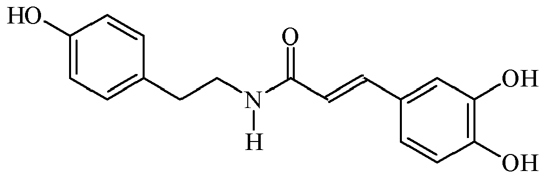

*N*-[2-(4-Hydroxyphenyl)ethyl]-3-(3,4-dihydroxyphenyl)-(2*Z*)-propensäureamid (cis-Caffeoyltyramin):

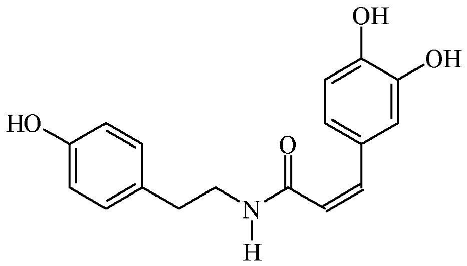

*N*-[2-(3,4-Dimethoxyphenyl)ethyl]-3-(3,4-dimethoxyphenyl)-(2*E*)-propensäureamid (trans-Rubenamin):

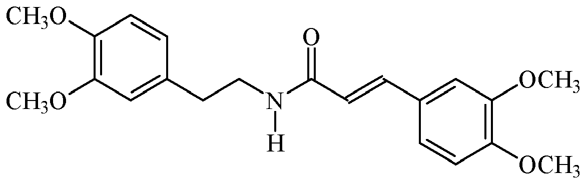

*N*-[2-(3,4-dimethoxyphenyl)ethyl]-3-(3,4-dimethoxy-phenyl)-(2*Z*)-propensäureamid (cis-Rubenamin):

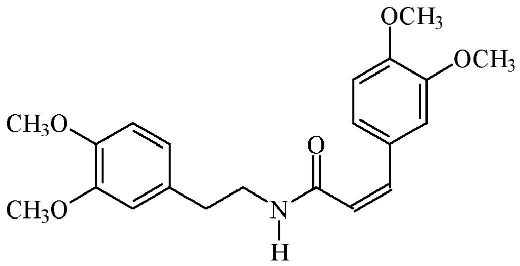

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenkliche pflanzliche Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*), Wasserpfefferextrakt (*Polygonum ssp.* ,insbesondere *Polygonum hydropiper*), Extrakte aus *Allium ssp*.(insbesondere Zwiebel und Knoblauchextrakte), Extrakte aus Rettich (*Raphanus ssp*.), Meerrettichextrakte (*Cochlearia armoracia*), Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Anacyclus ssp.,* insbesondere *Anacyclus pyrethrum*L.), Sonnenhutextrakte (*Echinaceae ssp*.), Extrakten aus Szechuan-Pfeffer (*Zanthoxylum ssp.,* insbesondere *Zanthoxylum piperitum*), Spilanthesextrakt (*Spilanthes ssp.,* insbesondere *Spilanthes acmella*)*,* Chiliextrakt (*Capsicum ssp.,* insbesondere *Capsicum frutescens*), Paradieskörner-Extrakt (*Aframomum ssp*.,insbesondere *Aframomum melegueta*[Rose] K. Schum.),Ingwerextrakt (*Zingiber ssp.,* insbesondere *Zingiber officinale*) und Galangaextrakt (*Kaempferia galanga* oder *Alpinia galanga*).

Eine besonders geeignete Substanz ist das aus dem Ingwerextrakt stammende Gingerol:

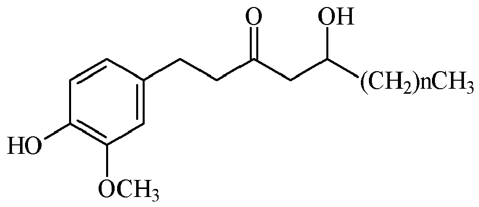

Einsetzbar ist auch N-Ethyl-p-menthan-3-carboxamid (N-Ethyl-5-Methyl-2-isopropylcyclohexancarboxamid):

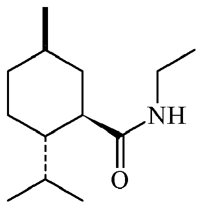

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Bevorzugte erfindungsgemäße remineralisierende Erzeugnisse sind dadurch gekennzeichnet, daß sie mindestens einen Scharfstoff aus der Gruppe der N-Alkyl-substituierte Amide von ungesättigten Carbonsäuren, vorzugsweise
a. 2E,6Z,8E-Decatriensäure-N-isobutylamid (Spilanthol) und/oder
b. 2E,4E,8Z-Decatriensäure-N-isobutylamid und/oder
c. 2E,7Z,9E-Undecatriensäure-N-isobutylamid und/oder
d. 2E,4Z-Decadiensäure-N-isobutylamid (cis-Pellitorin) und/oder
e. 2E,4E-Decadiensäure-N-isobutylamid (trans-Pellitorin) und/oder
f. Ferulasäure-N-Vanillylamid und/oder
g. *N*-[2-(4-Hydroxy-3-methoxyphenyl)ethyl]-3-(4-hydroxy-3-methoxy-phenyl)-(2*E*)-propensäureamid (trans-Feruloylmethoxytyramin) und/oder
h. *N*-[2- (4-Hydroxy-3-methoxyphenyl)ethyl]-3-(4-hydroxy-3-methoxy-phenyl)-(2*Z*)-propensäureamid (cis-Feruloylmethoxytyramin) und/oder
i. *N*-[2-(4-Hydroxy-3-methoxyphenyl)ethyl]-3-(4-hydroxy-3-methoxy-phenyl)-propansäureamid (Dihydroferuloylmethoxytyramin) und/oder
j. *N*-[2-(3,4-Dihydroxyphenyl)ethyl]-3-(4-hydroxy-3-methoxy-phenyl)-(2*E*)-propensäureamid (trans-Feruloyldopamin) und/oder
k. *N*-[2-(3,4-Dihydroxyphenyl)ethyl]-3-(4-hydroxy-3-methoxy-phenyl)-(2*Z*)-propensäureamid (cis- Feruloyldopamin) und/oder
l. *N*-[2-(4-Hydroxyphenyl)ethyl]-3-(3,4-dihydroxyphenyl)-(2*E*)-propensäureamid (trans-Caffeoyltyramin) und/oder
m. *N*-[2-(4-Hydroxyphenyl)ethyl]-3-(3,4-dihydroxyphenyl)-(2*Z*)-propensäureamid (cis-Caffeoyltyramin) und/oder
n. *N*-[2-(3,4-Dimethoxyphenyl)ethyl]-3-(3,4-dimethoxyphenyl)-(2*E*)-propensäureamid (trans- Rubenamin) und/oder
o. *N*-[2-(3,4-dimethoxyphenyl)ethyl]-3-(3,4-dimethoxy-phenyl)-(2*Z*)-propensäureamid (cis-Rubenamin)

### enthalten.

Zusätzlich zu den genannten Scharfstoffen oder an ihrer Stelle können auch weitere scharf schmeckende und/oder ein Gefühl von Wärme erzeugende Substanzen in die erfindungsgemäßen Erzeugnisse eingearbeitet werden.

Als besonders geeignet haben sich im Rahmen der vorliegenden Erfindung alkylsubstituierte Dioxane der Formel 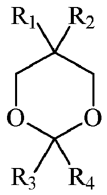 erwiesen, in der R1 und R2 unabhängig voneinander ausgewählt sind aus -H, -CH₃, -CH₂CH₃ und
R3 und R4 unabhängig voneinander ausgewählt sind aus -H, -CH₃, -CH₂CH₃, -CH₂CH₂CH₃, - CH(CH₃)₂.

Als weiterhin besonders geeignet haben sich im Rahmen der vorliegenden Erfindung Phenylester der Formel 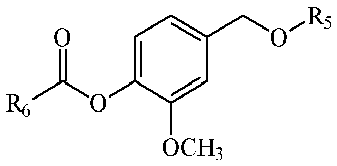 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Als weiterhin besonders geeignet haben sich im Rahmen der vorliegenden Erfindung Carvonacetale der Formel 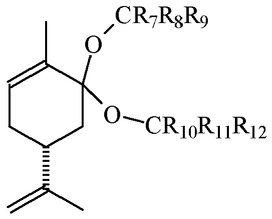 erwiesen, in der R7 bis R12 unabhängig voneinander ausgewählt sind aus -H, -CH₃, - CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, - CH(CH₃)CH₂CH₃, ₋C(CH₃)₃

Erfindungsgemäße Mittel können als Zahnpasten oder Zahncremes formuliert werden. Ein weiterer Gegenstand der vorliegenden Erfindung ist ein Verfahren zur Vorbeugung vor und Behandlung von Karies und/oder Bekämpfung von Halitosis und/oder zur Behandlung von Gingivitis oder Parodontitis, bei dem eine erfindungsgemäße Zubereitung auf eine Zahnbürste aufgetragen und mit dieser die Zähne geputzt werden.

Die erfindungsgemäßen Zusammensetzungen lassen sich auch als Mundspüllösungen oder Mundwässer formulieren. Ein weiterer Gegenstand der vorliegenden Erfindung ist ein Verfahren zur Vorbeugung vor und Behandlung von Karies und/oder Bekämpfung von Halitosis und/oder zur Behandlung von Gingivitis oder Parodontitis, bei dem eine erfindungsgemäße Zubereitung in Form einer Mundspüllösung in die Mundhöhle eingebracht und dort für einen Zeitraum von mindestens 10 Sekunden, vorzugsweise mindestens 20 Sekunden und insbesondere mindestens 45 Sekunden belassen wird.

Durch den Einsatz des 4-(3-Ethenyl-3,7-dimethyl-1,6-octadienyl)-phenols kann die Wirkung antibakterieller Substanzen synergistisch gesteigert werden. Ein weiterer Gegenstand der vorliegenden Erfindung ist daher die Verwendung von 4-(3-Ethenyl-3,7-dimethyl-1,6-octadienyl)-phenol zur Steigerung der Wirkung antibakterieller Verbindungen.

Bezüglich bevorzugter Ausführungsformen der erfindungsgemäßen Verwendungen und der erfindungsgemäßen Verfahren gilt mutatis mutandis das zu den erfindungsgemäßen Mitteln Gesagte.

### Beispiele:

### Mundspüllösungen (alle Angaben in Gew.-%):

| | **M1** | **M2** | **M3** | **M4** | **M5** | **M6** | **M7** | **M8** |
|---|---|---|---|---|---|---|---|---|
| | | 3 | 0 | 3 | 0 | 3 | 0 | 3 |
| Sorbitol 70% | 3,0 | | | | | | | |
| 4-(3-Ethenyl-3,7-dimethyl-1,6- | 0,1 | 0,3 | 0,1 | 0,3 | 0,1 | 0,3 | 0,3 | 0,3 |
| octadienyl)-phenol | | | | | | | | |
| Methylparaben | 0,2 | | | | | | | |
| Ethyparaben | | 0,2 | | | 0,1 | | | |
| Propylparaben | | | 0,2 | | 0,1 | | | |
| Butylparaben | | | | 0,2 | | | | |
| Silbercitrat | | | | | | 4 ppm Ag⁺ | | |
| Kupfer(II)sulfat | | | | | | | 5 ppm Cu⁺⁺ | |
| Zinksulfat-Heptahydrat | | | | | | | | 0,4 |
| Natriumfluorid | 0,05 | 0,05 | 0,05 | 0,05 | 0,05 | 0,045 | 0,045 | 0,045 |
| Saccharin Natrium | 0,03 | 0,03 | 0,03 | 0,03 | 0,03 | 0,03 | 0,03 | 0,03 |
| PEG-60 Hydrogenated Castor Oil | 0,86 | 0,86 | 0,86 | 0,86 | 0,86 | 0,86 | 0,86 | 0,86 |
| Aroma | 0,1 | 0,1 | 0,1 | 0,1 | 0,1 | 0,1 | 0,1 | 0,1 |
| Wasser, vollentsalzt | ad 100 | ad 100 | ad 100 | ad 100 | ad 100 | ad 100 | ad 100 | ad 100 |

Es wurden Zahnpasten folgender Zusammensetzung (alle Angaben in Gew.-%)hergestellt:

| | **E1** | **E2** | **E3** | **E4** | **E5** |
|---|---|---|---|---|---|
| Sorbit (70 % DAB) | 60 | 62,5 | 65 | 67,5 | 70,0 |
| Ethanol (96%) | - | - | - | - | 2,0 |
| Fällungskieselsäure : Sident 8 | 10,0 | 10,0 | 10,0 | 10,0 | 12,0 |
| Fällungskieselsäure : Sident 22S | 8,5 | 8,5 | 8,5 | 8,5 | - |
| Poliertonerde P10 feinst | - | 1,0 | - | - | - |
| Dinatriumphosphat, wasserfrei | 0,1 | 0,1 | 0,1 | 0,1 | 0,1 |
| Trinatriumphosphat, wasserfrei | 0,2 | 0,2 | 0,2 | 0,2 | - |
| Na-Monofluorophosphat Na2PO3F | - | - | 1,0 | 1,0 | 1,0 |
| Natriumfluorid | 0,32 | 0,32 | - | - | - |
| Polyethylenglycol (MG : 1500) | 1,5 | 1,5 | 1,5 | 1,5 | 1,5 |
| Titandioxid | 0,5 | 0,5 | 0,5 | 0,5 | - |
| Na-Laurylsulfat | 1,5 | 1,5 | 1,5 | 1,5 | 1,5 |
| Saccharin-Na | 0,1 | 0,1 | 0,1 | 0,1 | 0,1 |
| Xanthan | 0,4 | 0,4 | 0,4 | 0,4 | 0,4 |
| Aroma | 1,2 | 1,2 | 1,2 | 1,2 | 1,2 |
| 4-(3-Ethenyl-3,7-dimethyl-1,6-octadienyl)-phenol | 0,1 | 0,1 | 0,3 | 0,3 | 0,3 |
| Ethyparaben | 0,3 | | | | |
| Propylparaben | | 0,3 | | | |
| Silbercitrat | | | 4 ppm Ag⁺ | | |
| Kupfer(II)sulfat | | | | 5 ppm Cu⁺⁺ | |
| Zinksulfat-Heptahydrat | | | | | 0,3 |
| Wasser, vollentsalzt | ad 100 | ad 100 | ad 100 | ad 100 | ad 100 |

## Patentansprüche

1. Mund- und Zahnpflege- und -reinigungsmittel, enthaltend - bezogen auf sein Gewicht -
a) 0,001 bis 5 Gew.-% 4-(3-Ethenyl-3,7-dimethyl-1,6-octadienyl)-phenol und
b) 0,00001 bis 5 Gew.-% mindestens einer antibakteriellen Verbindung aus den Gruppen
h. der Benzoate und/oder
i. der Parabene und/oder
j. der Cu-Salze und/oder
k. der Ag-Salze und/oder
I. der Zn-Salze und/oder
m. Triclosan und/oder
n. Hexetidin.

2. Mund- und Zahnpflege- und -reinigungsmittel nach Anspruch 1, **dadurch gekennzeichnet, daß** es - bezogen auf sein Gewicht - 0,002 - 4 Gew.-%, vorzugsweise 0,003 - 3 Gew.-%, besonders bevorzugt 0,004 - 2 Gew.-%, außerordentlich bevorzugt 0,005 - 1 Gew.-% und insbesondere 0,01 bis 0,5 Gew.-% 4-(3-Ethenyl-3,7-dimethyl-1,6-octadienyl)-phenol enthält.

3. Mund- und Zahnpflege- und reinigungsmittel nach einem der Ansprüche 1 oder 2, **dadurch gekennzeichnet, daß** es - bezogen auf sein Gewicht - 0,01 - 4 Gew.-%, vorzugsweise 0,02 - 3 Gew.-%, besonders bevorzugt 0,05 - 2 Gew.-%, außerordentlich bevorzugt 0,1 - 1,5 Gew.-% und insbesondere 0,1 bis 1,0 Gew.-% p-Hydroxybenzoesäuremethyl-, -ethyl- oder propylester oder deren Mischungen enthält.

4. Mund- und Zahnpflege- und -reinigungsmittel nach einem der Ansprüche 1 bis 3, **dadurch gekennzeichnet, daß** es - bezogen auf sein Gewicht - 0,01 - 4 Gew.-%, vorzugsweise 0,02 - 3 Gew.-%, besonders bevorzugt 0,05 - 2 Gew.-%, außerordentlich bevorzugt 0,1 - 1,5 Gew.-% und insbesondere 0,1 bis 1,0 Gew.-% Parabene aus der Gruppe Methylparaben, Ethylparaben und deren Mischungen enthält.

5. Mund- und Zahnpflege- und -reinigungsmittel nach einem der Ansprüche 1 bis 4, **dadurch gekennzeichnet, daß** es - bezogen auf sein Gewicht - 0,00001 - 0,1 Gew.-%, vorzugsweise 0,00002 - 0,05 Gew.-%, besonders bevorzugt 0,00005 - 0,025 Gew.-%, außerordentlich bevorzugt 0,000075 - 0,015 Gew.-% und insbesondere 0,0001 bis 0,001 Gew.-% Silberionen, berechnet als Ag⁺, enthält.

6. Mund- und Zahnpflege- und -reinigungsmittel nach einem der Ansprüche 1 bis 5 **dadurch gekennzeichnet, daß** es - bezogen auf sein Gewicht - 0,00001 - 0,1 Gew.-%, vorzugsweise 0,00002 - 0,05 Gew.-%, besonders bevorzugt 0,00005 - 0,025 Gew.-%, außerordentlich bevorzugt 0,000075 - 0,015 Gew.-% und insbesondere 0,0001 bis 0,001 Gew.-% Kupferionen, berechnet als Cu²⁺, enthält.

7. Mund- und Zahnpflege- und -reinigungsmittel nach einem der Ansprüche 1 bis 6, **dadurch gekennzeichnet, daß** es - bezogen auf sein Gewicht - 0,01 - 5 Gew.-%, vorzugsweise 0,02 - 2,5 Gew.-%, besonders bevorzugt 0,03 - 1 Gew.-%, außerordentlich bevorzugt 0,04 - 0,5 Gew.-% und insbesondere 0,05 bis 0,2 Gew.-% Zinkionen, berechnet als Zn²⁺, enthält.

8. Mund- und Zahnpflege- und -reinigungsmittel nach einem der Ansprüche 1 bis 7, **dadurch gekennzeichnet, daß** es - bezogen auf sein Gewicht - 0,05 - 0,75 Gew.-%, vorzugsweise 0,06 - 0,5 Gew.-%, besonders bevorzugt 0,07 - 0,4 Gew.-%, außerordentlich bevorzugt 0,08 - 0,35 Gew.-% und insbesondere 0,1 bis 0,3 Gew.-% Triclosan enthält.

9. Mund- und Zahnpflege- und -reinigungsmittel nach einem der Ansprüche 1 bis 8, **dadurch gekennzeichnet, daß** es - bezogen auf sein Gewicht - 0,01 - 0,5 Gew.-%, vorzugsweise 0,02 - 0,4 Gew.-%, besonders bevorzugt 0,03 - 0,3 Gew.-%, außerordentlich bevorzugt 0,04 - 0,2 Gew.-% und insbesondere 0,05 bis 0,1 Gew.-% Hexetidin enthält.

10. Verwendung von 4-(3-Ethenyl-3,7-dimethyl-1,6-octadienyl)-phenol zur Steigerung der Wirkung antibakterieller Verbindungen.
